# EUROPEAN PATENT SPECIFICATION

(11) **EP 4 026 520 B1**
(45) Date of publication and mention of the grant of the patent: **11.10.2023**
(21) Application number: 22151008.4
(22) Date of filing: 11.01.2022
(51) Int. Cl.: A61F 2/38

(54) **CAM STABILIZED KNEE PROSTHESIS**
NOCKENSTABILISIERTE KNIEPROTHESE
PROTHÈSE DU GENOU STABILISÉE PAR CAME

(30) Priority: 12.01.2021 US 202163136353 P
(43) Date of publication of application: 13.07.2022
(73) Proprietor: Howmedica Osteonics Corporation, Mahwah, New Jersey 07430 (US)
(72) Inventor: COLLAZO, Carlos E., Old Greenwich, 06870 (US)
(74) Representative: Regimbeau

(56) References cited:
- GB-A- 2 296 443
- US-A1- 2010 016 977
- US-A1- 2012 029 649
- US-A1- 2020 069 432

## Description

### BACKGROUND

The present disclosure relates to orthopedics devices and, more particularly, to knee prostheses. The closest prior art is document US 2020/069432 A1, which defines the preamble of claim 1.

During articulation of a natural knee joint, flexion between the tibia and the femur takes place about a transverse axis while some relative rotation between the tibia and the femur occurs about a longitudinal axis. The rotation about the transverse axis through flexion and extension is not as simple as that of a hinge of a door in which its rotational axis remains stationary. Instead, the transverse axis of the knee moves in the anteroposterior direction during flexion and extension. In this regard, as the knee joint flexes from an extended position, the center of contact between the femur and tibial plateau moves posteriorly. This is known as femoral roll-back which is generally facilitated by the posterior cruciate ligament. Femoral roll-back is the knee's natural mechanism for maximizing the range of possible flexion and for also maximizing efficiency of the extensor mechanism.

Total knee arthroplasty commonly involves the sacrifice of the anterior and posterior cruciate ligaments. The cruciate ligaments, in addition to providing for femoral roll-back, also facilitate the natural internal-external rotation of the tibia relative to the femur and resist paradoxical movement between these bones during certain activities, such as those involving a heel strike. Since the removal of these ligaments has the attendant consequence of removing their kinematic functionality, it is often the objective of total knee prostheses to gain some measure of functionality back.

Conventional knee prostheses often utilize a cam and post mechanism to account for the elimination of the cruciate ligaments. Some tibial baseplates even include a spherical member at the end of its post to articulate with a spherical cavity in a femoral component to help facilitate internal-external rotation. However, in prostheses such as these, the post member must have a considerable height to articulate with the femoral component which can result in the post bearing significant cyclic loads. Moreover, femoral roll-back may be precluded by being constrained.

Although several knee prostheses have been developed over the years, improvements are still possible. A need exists for knee prostheses capable of more closely imitating the natural knee.

### BRIEF SUMMARY OF THE DISCLOSURE

A total knee prosthesis according to the invention is defined in claim 1. Embodiments of the total knee prosthesis are defined in the dependent claims.

A total knee prosthesis described in this disclosure includes a femoral component and a tibial component. The femoral component includes a spherical member and a cam member disposed within an intercondylar notch. The tibial component includes a post defining a recess thereon. The recess is sized to receive the spherical member. The spherical member is adapted to articulate with the recess of the post, thus allowing the femoral component to articulate relative to the tibial component. The spherical member may rotate within the recess until a degree of flexion in which the cam member contacts the post of the tibial component, wherein further flexion may cause the spherical member to translate within the recess. The articulation between the spherical member and the post occurs at a distal portion of the spherical member and the cam member contacts the post at a height such that a moment force may be applied to the tibial most that may not require additional support to withstand.

The total knee prosthesis includes a femoral component having an anterior flange, a first femoral condyle, a second femoral condyle, a spherical member and a cam member. The first and second femoral condyles extend from the anterior flange and at least partially defining an intercondylar recess therebetween. The spherical member and cam member are positioned within the intercondylar recess such that the cam member is offset from the spherical member in a posterior direction. The total knee prosthesis further includes a tibial component having a bone contact side and an articular side. The articular side has first and second tibial condyles and a post extending therefrom. The post has a post recess extending into an end thereof and is sized to receive the spherical member. The post recess defines an anterior spherical portion and a posterior runout portion. The anterior spherical portion has a radius of curvature in a sagittal plane extending from the post. The posterior runout portion is linear and tangent to the anterior spherical portion in the sagittal plane.

The tibial component may include a baseplate and an insert connectable to the baseplate. The baseplate may define the bone contact side of the tibial component. The insert may define the articular side of the femoral component. The insert may have the post and first and second tibial condyles. The insert may include a base configured to connect to the tibial baseplate. The post may extend from the base. The post and the base may form a monolithic structure. The baseplate may include a plurality of pegs extending from the bone contact side.

The posterior runout portion may define a posterior extent of the recess. The anterior spherical portion may define an anterior extent of the recess. The post may have a sidewall extending in an inferior-superior direction and defining a perimeter thereof. The spherical member may articulate with the anterior spherical portion and posterior runout portion. The cam may articulate with a posterior aspect of the sidewall through flexion and extension of the total knee prosthesis. The cam may engage the posterior aspect of the sidewall at 90 degrees of flexion. The spherical member may articulate with the spherical portion at - 15 to 90 degrees of flexion and may articulate with the runout portion at 90 to 150 degrees of flexion. The spherical member may both rotate and translate in an anteroposterior direction relative to the spherical and runout portions of the post cavity. The spherical member may have a radius of curvature of 7.6 mm. The radius of curvature of the spherical portion may be 7.6 mm.

The femoral component may be adapted to define a first position in which the spherical member is positioned within the anterior spherical portion of the post and the cam member does not contact the post. The femoral component may be adapted to define a second position in which the spherical member is positioned within the anterior spherical portion of the post and the cam member contacts that post. The femoral component may be adapted to define a third position in which the spherical member is positioned within the posterior runout portion and the cam member contacts the post. The femoral component may be adapted to define a fourth position in which the spherical member does not contact the recess of the post and the cam member contacts the post. A distal portion of the spherical member may contact and articulate with the recess of the post of the tibial component. The posterior runout portion may include medial and lateral portions curving in a superior direction forming a cylindrical shape within the recess. The radius of curvature of the medial and lateral portions of the posterior runout portion may be substantially equal to the radius of curvature of the spherical member.

### BRIEF DESCRIPTION OF THE DRAWINGS

FIG. 1 is a cross-sectional side view of a knee prosthesis taken along a midline thereof and according to an embodiment of the disclosure.
FIGS. 2A-B are perspective superior and inferior views, respectively, of a femoral component of the knee prosthesis of FIG. 1.
FIG. 3A is a perspective superior view of a tibial component of the knee prosthesis of FIG. 1.
FIG. 3B is perspective superior view of an insert of the tibial component of FIG. 3A
FIG. 3C is a perspective side view of the insert of FIG. 3B.
FIGS. 4A-E illustrate the knee prosthesis of FIG. 1 in first, second, third, fourth and fifth positions of flexion, respectively.

### DETAILED DESCRIPTION

As used herein, when referring to bones or other parts of the body, the term "proximal" means closer to the heart and the term "distal" means more distant from the heart. The term "inferior" means towards the feet and the term "superior" means towards the head. The term "medial," when used in connection with a component or a patient's anatomy, refers to a side or region facing the center of the patient. The term "lateral," when used in connection with a component or a patient's anatomy, refers to a side or region facing toward the patient's side, i.e., away from the center of the patient. The term "anterior," when used in connection with a component or a patient's anatomy, refers to a side or region facing the front of the patient. The term "posterior," when used in connection with a component or a patient's anatomy, refers to a side or region facing the rear of the patient. As used herein, the terms "substantially," "generally," "approximately," and "about" are intended to mean that slight deviations from absolute are included within the scope of the term so modified.

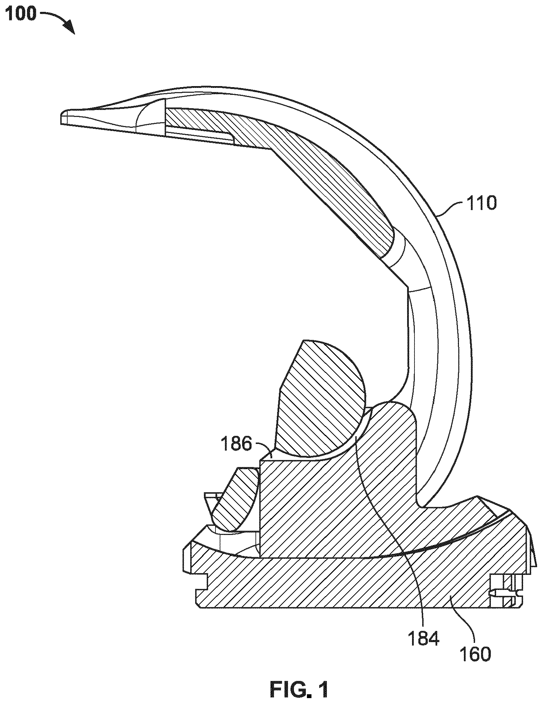

FIG. 1 illustrates an embodiment of a stabilized knee prosthesis 100 for replacing a natural knee joint. Knee prosthesis 100 includes a femoral component 110 and a tibial component 160. In operation, femoral component 110 may articulate relative to tibial component 160. Femoral component 110 is adapted to be attached to a distal end of a femur, whereas tibial component 150 is adapted to be attached to a proximal end of a tibia in a well-known manner. Both the distal end of the femur and the proximal end of the tibia may be resected or prepared before implantation of knee prosthesis 100. In some embodiments, knee prosthesis 100 is wholly or partly made of a substantially rigid material, such as titanium, titanium alloy, chrome-cobalt alloy, cobalt-chrome-molybdenum alloys (e.g., cobalt-chromium-molybdenum alloy sold under the trademark Vitallium^{®}), polyethylene, polyether ether ketone (PEEK), or any suitable metal or polymer.

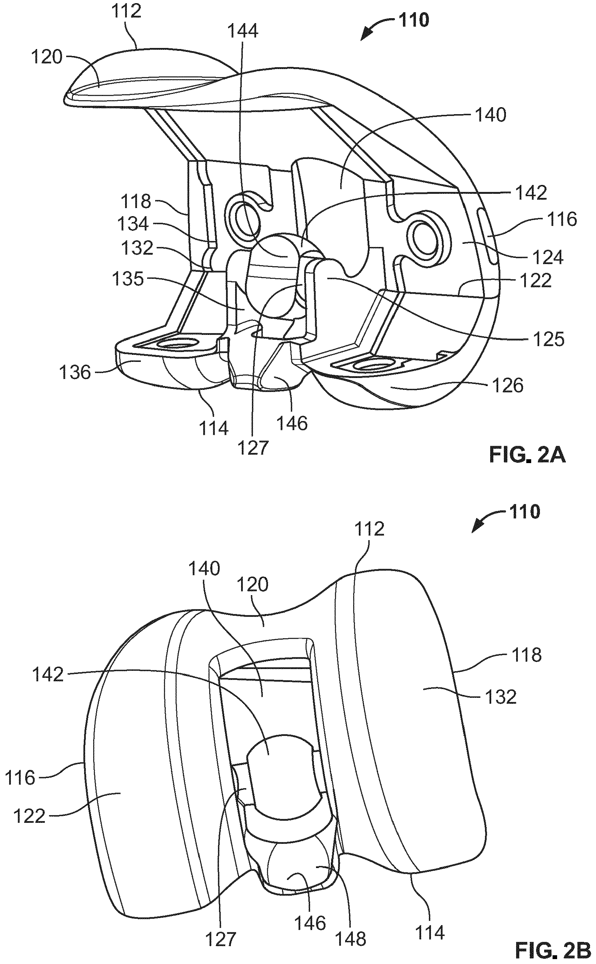

FIGS. 2A-B illustrate femoral component 110 in greater detail according to an embodiment of the disclosure. Femoral component 110 extends from an anterior end 112 to a posterior end 114 defining a length of the femoral component 110. Femoral component 110 further extends from a medial edge 116 to a lateral edge 118 defining a width of the femoral component 110. Femoral component 110 includes an anterior flange 120 which extends along the width of femoral component 110 and defines an anterior and superior terminal end of the femoral component. Femoral component 110 further includes a medial condyle 122 that extends from flange 120 on a medial side of the femoral component. More specifically, medial condyle 122 has a central portion 124 that extends from flange 120 generally in a posterior direction from anterior flange 120, and a posterior portion 126 which extends from central portion 124 generally in a posterior and superior direction. Femoral component 110 further includes a lateral condyle 132 that extends from anterior flange 120 on a lateral side of femoral component 110. Lateral condyle 132 has a central portion 134 that extends from flange 120 generally in a posterior direction, and a posterior portion 136 extending from central portion 134 generally in a posterior and superior direction. It is noted that the posterior portions 126, 136 of the medial and lateral condyles 122, 132 respectively define posterior and superior terminal ends of femoral component 110 opposite that of anterior flange 120.

Medial condyle 122 and lateral condyle 132 define an intercondylar notch 140 therebetween. Anterior flange 120 extends from anterior end 112 to the anterior-most point of intercondylar notch 140. In this regard, anterior flange 120 defines an anterior extent of intercondylar notch 140. Medial and lateral condyles 122, 132 begin extending from anterior flange 120 at the length of femoral component 110 where intercondylar notch 140 begins. That is, anterior flange 120 ends and condyles 122, 132 begin along an axis extending across the width of femoral component 110 tangential to the anterior-most point of intercondylar notch 140.

Femoral component 110 further includes a spherical member 142 and a cam member 146 positioned within the intercondylar notch 140. Spherical member 142 is connected to condyles 122, 132 via flanges 125, 135 that extend superiorly from an inner edges of condyles 122, 132, respectively, and a horizontal post 127 that extends from the flanges 125, 135. Spherical member 142 is positioned on post 127 so that its spherical curvature is positioned within the intercondylar notch 140 and pointing in an inferior direction. Spherical member 142 is also positioned superior to the condyles 122, 132. Spherical member 142, as shown, is a partial sphere such that a superior side defines a planar surface 144. This allows femoral component 110 to be placed directly against a planar resected surface of a femur. In some examples, the spherical member 142 may have a radius of curvature of 5-10 millimeters. In one particular example, such as the example depicted in the figures, spherical member 142 may have a radius of curvature of 7.6 millimeters.

The cam member 146, as in the example shown, may be offset from the spherical member 142 in a posterior direction. In other words, the cam member 146 may be disposed within the intercondylar notch 140 nearer the posterior terminal end formed by the medial and lateral condyles 122, 132 than the spherical member 142. Spherical member 142 and cam member 146 may contact and articulate with components of tibial component 160 when knee prosthesis 100 is in an assembled configuration, as described in greater detail below. In this regard, cam member 146 includes a concavely curved anterior face that matches the convex posterior curvature of post 180 of tibial component 160.

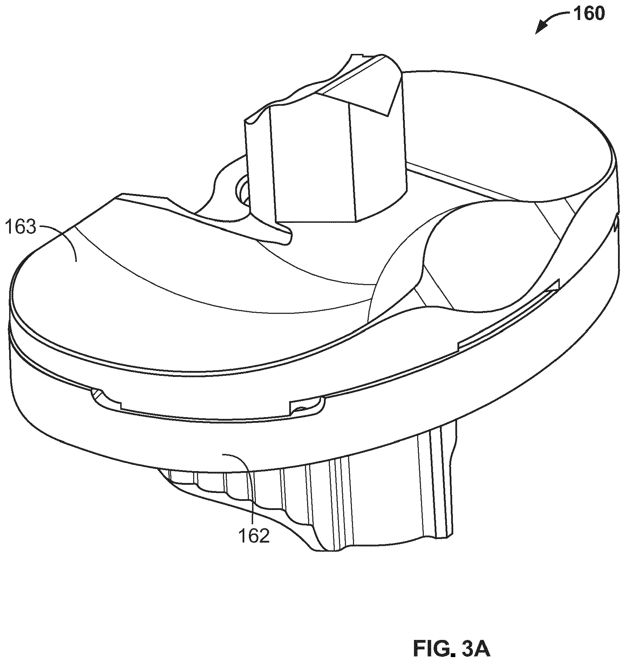

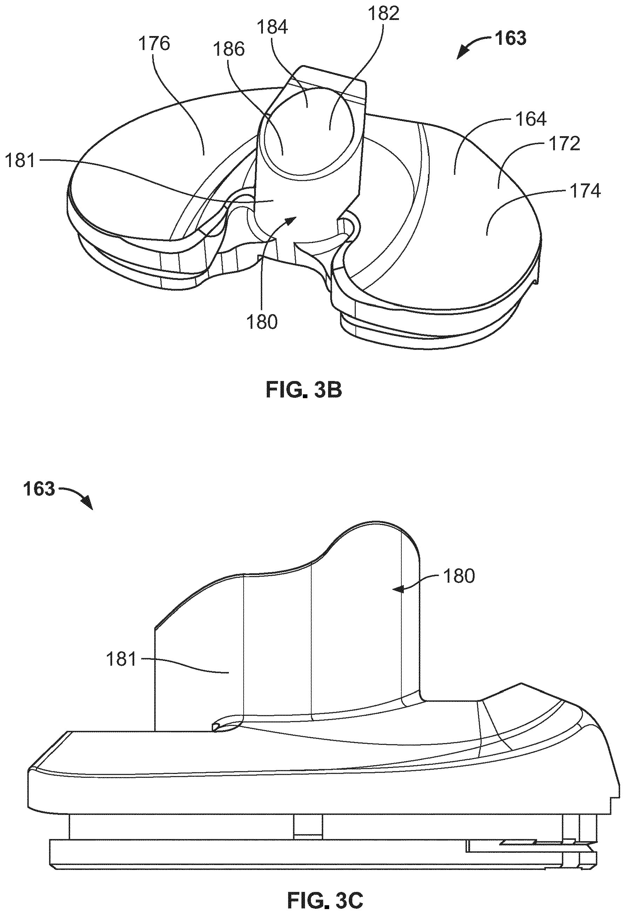

FIG. 3A illustrates tibial component 160 in greater detail according to an embodiment of the disclosure. Tibial component 160 generally includes a baseplate 162 coupled to an insert 163. Baseplate 162 defines a bone contact side configured to contact the tibia when in an implanted configuration. The baseplate may include a plurality of pegs (not shown) extending from the baseplate on the bone contact side to anchor the tibial component into a bone when in the implanted configuration.

FIGS. 3B-C illustrate insert 163 in greater detail. Insert 163 includes a base 164. The base 164 defines an articular side 172 and a baseplate connection side. Base 164 includes a medial tibial condyle 174 and a lateral tibial condyle 176 on articular side 172. Medial and lateral tibial condyles 174, 176 are adapted to receive and support medial and lateral femoral condyles 122, 132, respectively. Femoral condyles 122, 132 may articulate with respective tibial condyles 174, 176. Insert 163 further includes a post 180 extending from the base 164 of insert 163 on articular side 172. In the illustrated embodiment, post 180 and base 164 are monolithic; however, it is also contemplated that post 180 and base 164 may be modular components configured to couple and decouple from each other. Post 180 defines a sidewall 181 which extends from base 164 of insert 163 in a superior direction, the sidewall 181 defining a perimeter of the post 180. Post 180 has an ovular shape such that its posterior and anterior ends are convexly curved. When knee prosthesis 100 is in an assembled configuration, cam member 146 of femoral component 110 is configured to articulate with a posterior aspect of sidewall 181 of post 180 through flexion and extension of knee prosthesis 100.

Post 180 defines a recess 182 extending into a superior end of the post 180 (*i*.*e*., an end opposite the end coupled to articular side 172). The recess 182 is sized to fit spherical member 142. The post recess 182 further defines an anterior spherical portion 184 and a posterior runout portion 186. The anterior spherical portion 184 has a radius of curvature in a sagittal plane extending through the post 180, as best shown in FIG. 1. In some examples, anterior spherical portion 184 may have a radius of curvature complimentary to that of spherical member 142. In this regard, spherical portion 184 may have the same radius of curvature, such as, for example, 5-10 millimeters, or more specifically 7.6 millimeters as in the example depicted. The anterior spherical portion 184 may define an anterior extent of the post recess 182. The posterior runout portion 186 is linear and tangent to the anterior spherical portion 184 in the sagittal plane, as best shown in FIG. 1. The posterior runout portion 186 may define a posterior extent of the post recess 182. Posterior runout portion 186 may include medial and lateral portions extending in a superior direction so as to form a cylindrical shape. Such a cylindrical shape may be sized to fit the spherical member 142 (e.g., the cylindrical posterior runout portion have a radius of curvature substantially equal to the radius of curvature of the spherical member) to allow translation in a single direction. When knee prosthesis 100 is in an implanted configuration, spherical member 142 of femoral component 110 is configured to articulate with anterior spherical portion 184 and posterior runout portion 186 of post recess 182. Spherical member 142 may rotate and translate in an anteroposterior direction relative to anterior spherical portion 184 and posterior runout portion 186, as described below in further detail. In this regard, prosthesis 100 provides for femoral roll-back.

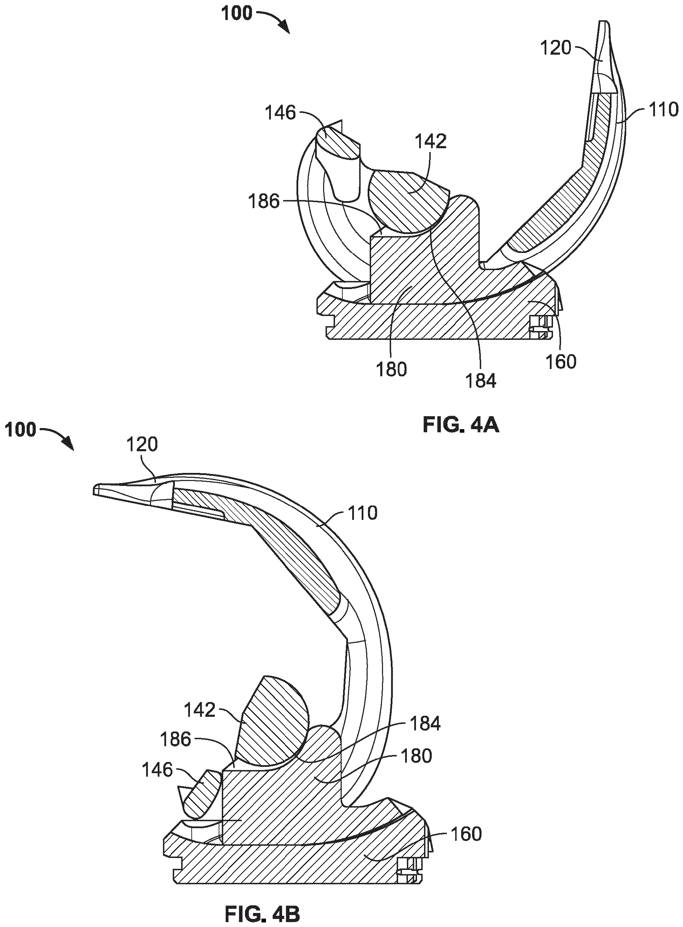

As seen in FIGS. 4A-E, knee prosthesis 100 substantially mimics the kinematics of the natural knee through flexion and extension. When knee prosthesis 100 is implanted in a patient, femoral component 110 is in contact with and articulates with respect to tibial component 160. In this regard, femoral condyles 122, 132 contact and articulate with tibial condyles 174, 176. Also, post 180 extends into intercondylar notch 140 so that spherical member 142 is positioned within cavity 182. When prosthesis 100 is articulated through a range of motion in flexion and extension spherical member 142 articulates with spherical portion 184 and runout portion 186. Moreover, in greater degrees of flexion, such as 90 degrees and beyond, cam member 146 contacts and articulates with post 180. FIGS. 4A-E illustrate knee prosthesis 100 in various positions, each position representing the configuration of knee prosthesis 100 according to a degree of flexion of the knee.

Knee prosthesis 100 is shown in a first position in FIG. 4A in which the knee is fully extended (*i.e*., zero degrees of flexion). In the first position, spherical member 142 is located in anterior spherical portion 184 of post recess 182. In the first position, cam member 146 does not contact post 180. Further, anterior flange 120 is positioned anteriorly relative to other components of knee prosthesis 100 and extends in a superior direction. As knee prosthesis 100 is flexed from the first position, spherical member 142 articulates with anterior spherical portion 184 of post recess 182 and cam member 146 rotates with femoral component 110 toward post 180, as illustrated in subsequent figures. While in this position, spherical member 142 and spherical portion 184 of recess 182 not only allows femoral component to rotate in flexion and extension, but also with internal/external rotation.

Knee prosthesis 100 is shown in a second position in FIG. 4B in which the knee prosthesis 100 is flexed at ninety degrees of flexion. In the second position, spherical member 142 is disposed in anterior spherical portion 184 of post recess 182 similar to the first position. Upon transitioning from the first position to the second position, spherical member 142 articulates with anterior spherical portion 184 of post recess 182. In some examples, spherical member 142 may articulate within anterior spherical portion 184 between -15 degrees of flexion (*i.e*., hyperextension) and 90 degrees of flexion. In the second position, femoral component 110 has articulated and rotated relative to the first position, such that the anterior flange 120 is positioned superior to the other components of knee prosthesis 100 extending in a posterior direction, and cam member 146 contacts the sidewall 181 of post 180. Due to such contact between the cam member 146 and the sidewall 181 of post 180, additional flexion of the knee beyond ninety degrees shown in the second position may cause translation of the spherical member 142 within the post recess 182, as will be shown in subsequent figures.

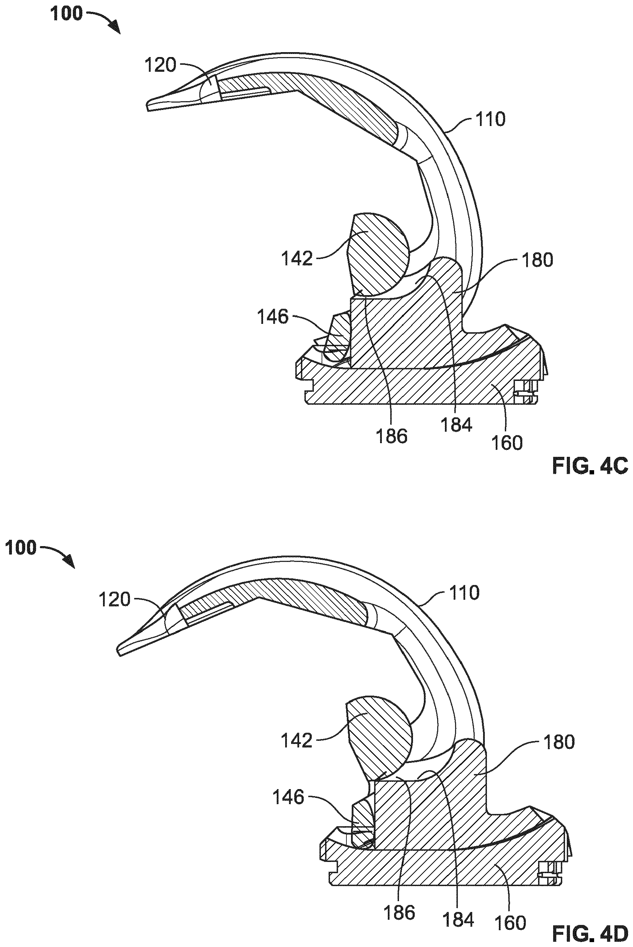

FIGS. 4C-E illustrate knee prosthesis 100 in various positions greater than ninety degrees of flexion. For example, FIG. 4C illustrates knee prosthesis 100 in a third position in which anterior flange 120 extends in a posterior and inferior direction. Upon transitioning from the second position to the third position, spherical member 142 translates within the post recess 182. In the third position, cam member 146 remains in contact and articulates with sidewall of post 180, and spherical member 142 is positioned within the posterior runout portion 186 of post recess 182. In some examples, spherical member 142 may articulate with posterior runout portion 186 of post recess 182 between 90 degrees of flexion and 150 degrees of flexion.

FIG. 4D illustrates knee prosthesis 100 in a fourth position in which anterior flange 120 extends further in a posterior and inferior direction relative to the third position. Further in the fourth position, spherical member 142 is positioned further in the posterior direction along posterior runout portion 186 relative to the third position due to articulation of the spherical member 142 upon transitioning from the third position to the fourth position. In the fourth position, cam member 146 remains in contact and articulates with sidewall 181 of post 180.

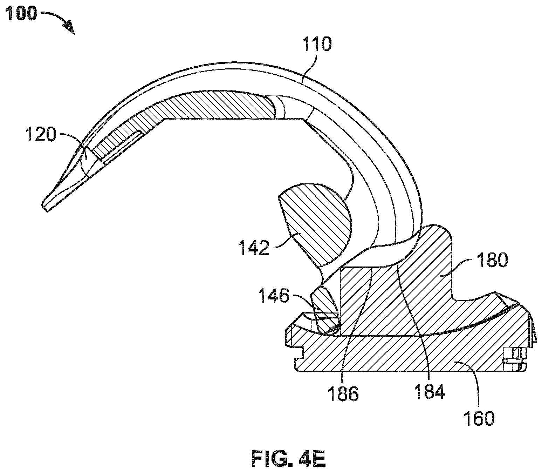

FIG. 4E illustrates knee prosthesis 100 in a fifth position in which anterior flange 120 extends still further in a posterior and inferior direction relative to the fourth position. In the fifth position, cam member 146 remains in contact with the sidewall 181 of post 180, and spherical member 142 is displaced from post 180 and no longer in contact with post recess 182 upon transitioning from the fourth position to the fifth position. It should be understood that knee prosthesis 100 may assume any and each of the above-described positions as knee prosthesis 100 transitions from full extension (as shown in FIG. 4A) to full flexion and *vice versa.*

The structure of the knee prosthesis 100 allows for articulation between the femoral condyles 122, 132 and the tibial condyles 174, 176 as well as between post 180 and both cam member 146 and spherical member 142. This articulation operates to provide femoral roll-back of femoral component 110, particularly as demonstrated between FIGS. 4C and 4E. In this regard, contact between the condyles can translate posteriorly *(i.e*., roll-back) in a similar manner to that of a natural knee. This is facilitated by the similar kinematics of spherical member 142 in which spherical member 142 rolls back from spherical portion 184 to runout portion 186. The articulation of cam member 146 on the posterior aspect of post 180 further facilitates the roll-back articulation.

In addition to these kinematics, the positioning of the spherical member 142 within intercondylar notch 140 of femoral component 110 minimizes the length of the post 180 needed for this sort of articulation. This is because the spherically curved surface of spherical member 142 is positioned inferior with respect to its center of curvature rather than superior to such center of curvature.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. A total knee prosthesis (100), comprising:
a femoral component (110) having an anterior flange (120), a first femoral condyle (122), a second femoral condyle (132) and a spherical member (142), the first and second femoral condyles extending from the anterior flange and at least partially defining an intercondylar recess (140) therebetween; and
a tibial component (160) having a bone contact side and an articular side (172), the articular side having first (174) and second (176) tibial condyles and a post (180) extending therefrom, the post having a post recess (182) extending into an end thereof and sized to receive the spherical member, the post recess defining an anterior spherical portion (184) and a posterior runout portion (186), the anterior spherical portion having a radius of curvature in a sagittal plane extending from the post, and the posterior runout portion being linear and tangent to the anterior spherical portion in the sagittal plane, **characterised in that** the femoral component further comprises a cam member (146),
wherein the spherical member and the cam member are positioned within the intercondylar recess such that the cam member is offset from the spherical member in a posterior direction.

2. The total knee prosthesis of claim 1, wherein the tibial component includes a baseplate (162) and an insert (163) connectable to the baseplate, the baseplate defining the bone contact side of the tibial component, the insert defining the articular side of the femoral component and having the post and first and second tibial condyles.

3. The total knee prosthesis of claim 2, wherein the insert includes a base (164) configured to connect to the tibial baseplate and the post extends from the base.

4. The total knee prosthesis of claim 3, wherein the post and the base form a monolithic structure.

5. The total knee prosthesis of any one of claims 2-4, wherein the baseplate includes a plurality of pegs extending from the bone contact side.

6. The total knee prosthesis of any one of claims 1-5, wherein the posterior runout portion defines a posterior extent of the recess and the anterior spherical portion defines an anterior extent of the recess.

7. The total knee prosthesis of any one of claims 1-6, wherein the post has a sidewall (181) extending in an inferior-superior direction and defining a perimeter thereof.

8. The total knee prosthesis of claim 7, wherein the spherical member articulates with the anterior spherical portion and posterior runout portion and the cam articulates with a posterior aspect of the sidewall through flexion and extension of the total knee prosthesis.

9. The total knee prosthesis of claim 8, wherein the cam engages the posterior aspect of the sidewall at 90 degrees of flexion.

10. The total knee prosthesis of any one of claims 8-9, wherein the spherical member articulates with the spherical portion at -15 to 90 degrees of flexion and articulates with the runout portion at 90 to 150 degrees of flexion.

11. The total knee prosthesis of any one of claims 8-10, wherein the spherical member both rotates and translates in an anteroposterior direction relative to the spherical and runout portions of the post cavity.

12. The total knee prosthesis of any one of claims 1-11, wherein the femoral component is adapted to define a first position in which the spherical member is positioned within the anterior spherical portion of the post and the cam member does not contact the post.

13. The total knee prosthesis of any one of claims 1-12, wherein the femoral component is adapted to define a second position in which the spherical member is positioned within the anterior spherical portion of the post and the cam member contacts that post.

14. The total knee prosthesis of any one of claims 1-13, wherein the femoral component is adapted to define a third position in which the spherical member is positioned within the posterior runout portion and the cam member contacts the post.

15. The total knee prosthesis of any one of claims 1-14, wherein the femoral component is adapted to define a fourth position in which the spherical member does not contact the recess of the post and the cam member contacts the post.

## Patentansprüche

1. Eine totale Knieprothese (100), die Folgendes umfasst:
eine Femurkomponente (110) mit einem vorderen Flansch (120), einem ersten Femurkondylus (122), einem zweiten Femurkondylus (132) und einem kugelförmigen Element (142), wobei sich der erste und der zweite Femurkondylus von dem vorderen Flansch aus erstrecken und zumindest teilweise eine interkondyläre Aussparung (140) dazwischen bilden; und
eine Tibiakomponente (160) mit einer Knochenkontaktseite und einer Gelenkseite (172), wobei die Gelenkseite eine erste (174) und eine zweite (176) Tibiakondyle und einen sich davon erstreckenden Pfosten (180) aufweist, wobei der Pfosten eine Pfostenaussparung (182) aufweist, die sich in ein Ende davon erstreckt und so bemessen ist, dass sie das kugelförmige Element aufnimmt, wobei die Pfostenaussparung einen vorderen kugelförmigen Abschnitt (184) und einen hinteren Auslaufabschnitt (186) definiert, wobei der vordere sphärische Abschnitt einen Krümmungsradius in einer sagittalen Ebene aufweist, die sich von dem Pfosten aus erstreckt, und der hintere Auslaufabschnitt linear und tangential zu dem vorderen sphärischen Abschnitt in der sagittalen Ebene ist, **dadurch gekennzeichnet, dass** die femorale Komponente ferner ein Nockenelement (146) umfasst, wobei das sphärische Element und das Nockenelement innerhalb der interkondylären Aussparung derart positioniert sind, dass das Nockenelement von dem sphärischen Element in einer hinteren Richtung versetzt ist.

2. Knietotalprothese nach Anspruch 1, wobei die Tibiakomponente eine Grundplatte (162) und einen Einsatz (163) umfasst, der mit der Grundplatte verbunden werden kann, wobei die Grundplatte die Knochenkontaktseite der Tibiakomponente definiert, der Einsatz die Gelenkseite der Femurkomponente definiert und den Pfosten sowie den ersten und zweiten Tibiakondylen aufweist.

3. Knietotalprothese nach Anspruch 2, wobei der Einsatz eine Basis (164) aufweist, die so konfiguriert ist, dass sie mit der Tibiabasisplatte verbunden werden kann, und der Pfosten von der Basis ausgeht.

4. Knietotalprothese nach Anspruch 3, wobei der Pfosten und die Basis eine monolithische Struktur bilden.

5. Knietotalprothese nach einem der Ansprüche 2 bis 4, wobei die Grundplatte eine Vielzahl von Zapfen aufweist, die von der Knochenkontaktseite ausgehen.

6. Knietotalprothese nach einem der Ansprüche 1 bis 5, wobei der hintere Auslaufabschnitt eine hintere Ausdehnung der Aussparung definiert und der vordere kugelförmige Abschnitt eine vordere Ausdehnung der Aussparung definiert.

7. Knietotalprothese nach einem der Ansprüche 1 bis 6, wobei der Pfosten eine Seitenwand (181) aufweist, die sich in einer Richtung von unten nach oben erstreckt und einen Umfang davon definiert.

8. Knietotalprothese nach Anspruch 7, wobei das kugelförmige Element mit dem vorderen kugelförmigen Teil und dem hinteren Auslaufabschnitt gelenkig verbunden ist und der Nocken mit einem hinteren Aspekt der Seitenwand durch Beugung und Streckung der Knietotalprothese gelenkig verbunden ist.

9. Knietotalprothese nach Anspruch 8, bei der die Nocke bei 90 Grad Beugung an der hinteren Seite der Seitenwand angreift.

10. Knietotalprothese nach einem der Ansprüche 8-9, wobei das kugelförmige Element mit dem kugelförmigen Teil bei -15 bis 90 Grad Beugung und mit dem Auslaufabschnitt bei 90 bis 150 Grad Beugung gelenkig verbunden ist.

11. Knietotalprothese nach einem der Ansprüche 8 bis 10, wobei das kugelförmige Element in einer anteroposterioren Richtung relativ zu den kugelförmigen und auslaufenden Teilen des Pfostenaussparung sowohl rotiert als auch verschoben wird.

12. Knietotalprothese nach einem der Ansprüche 1 bis 11, wobei die Femurkomponente so ausgelegt ist, dass sie eine erste Position definiert, in der das kugelförmige Element innerhalb des vorderen kugelförmigen Teils des Pfostens positioniert ist und das Nockenelement den Pfosten nicht berührt.

13. Knietotalprothese nach einem der Ansprüche 1 bis 12, wobei die Femurkomponente so beschaffen ist, dass sie eine zweite Position definiert, in der das kugelförmige Element innerhalb des vorderen kugelförmigen Teils des Pfostens positioniert ist und das Nockenelement diesen Pfosten berührt.

14. Knietotalprothese nach einem der Ansprüche 1 bis 13, wobei die Femurkomponente so ausgelegt ist, dass sie eine dritte Position definiert, in der das kugelförmige Element innerhalb des hinteren Auslaufteils positioniert ist und das Nockenelement den Pfosten berührt.

15. Knietotalprothese nach einem der Ansprüche 1 bis 14, wobei die Femurkomponente so ausgelegt ist, dass sie eine vierte Position definiert, in der das kugelförmige Element die Aussparung des Pfostens nicht berührt und das Nockenelement den Pfosten berührt.

## Revendications

1. Prothèse totale de genou (100), comprenant :
un composant fémoral (110) comportant un rebord antérieur (120), un premier condyle fémoral (122), un second condyle fémoral (132) et un élément sphérique (142), les premier et second condyles fémoraux s'étendant à partir du rebord antérieur et définissant au moins partiellement un renfoncement intercondylaire (140) entre eux ; et
un composant tibial (160) ayant un côté de contact avec l'os et un côté articulaire (172), le côté articulaire ayant un premier (174) et un second (176) condyles tibiaux et un tenon (180) s'étendant à partir de celui-ci, le tenon ayant un renfoncement de tenon (182) s'étendant dans une de ses extrémités et dimensionné pour recevoir l'élément sphérique, le renfoncement de tenon définissant une partie sphérique antérieure (184) et une partie de sortie postérieure (186), la partie sphérique antérieure ayant un rayon de courbure dans un plan sagittal s'étendant à partir du tenon, et la partie de sortie postérieure étant linéaire et tangente à la partie sphérique antérieure dans le plan sagittal, **caractérisée en ce que** le composant fémoral comprend en outre un élément de came (146), dans lequel l'élément sphérique et l'élément de came sont positionnés dans le renfoncement intercondylaire de telle sorte que l'élément de came est décalé par rapport à l'élément sphérique dans une direction postérieure.

2. La prothèse totale de genou de la revendication 1, dans laquelle le composant tibial comprend une plaque de base (162) et un insert (163) connectable à la plaque de base, la plaque de base définissant le côté de contact avec l'os du composant tibial, l'insert définissant le côté articulaire du composant fémoral et comportant le tenon et les premier et second condyles tibiaux.

3. La prothèse totale de genou de la revendication 2, dans laquelle l'insert comprend une base (164) configurée pour se connecter à la plaque de base tibiale et le tenon s'étend à partir de la base.

4. La prothèse totale de genou de la revendication 3, dans laquelle le tenon et la base forment une structure monolithique.

5. La prothèse totale de genou de l'une des revendications 2 à 4, dans laquelle la plaque de base comprend une pluralité de plots s'étendant à partir du côté de contact avec l'os.

6. La prothèse totale de genou de l'une des revendications 1 à 5, dans laquelle la partie postérieure du renfoncement définit une étendue postérieure de l'évidement et la partie sphérique antérieure définit une étendue antérieure du renfoncement.

7. La prothèse totale de genou de l'une des revendications 1 à 6, dans laquelle le tenon a une paroi latérale (181) s'étendant dans une direction inférieure-supérieure et définissant son périmètre.

8. La prothèse totale de genou de la revendication 7, dans laquelle l'élément sphérique s'articule avec la partie sphérique antérieure et la partie de sortie postérieure et la came s'articule avec un aspect postérieur de la paroi latérale lors de la flexion et de l'extension de la prothèse totale de genou.

9. La prothèse totale de genou de la revendication 8, dans laquelle la came s'engage dans l'aspect postérieur de la paroi latérale à 90 degrés de flexion.

10. La prothèse totale de genou de l'une des revendications 8 à 9, dans laquelle l'élément sphérique s'articule avec la partie sphérique entre -15 et 90 degrés de flexion et s'articule avec la partie de sortie entre 90 et 150 degrés de flexion.

11. La prothèse totale de genou de l'une des revendications 8 à 10, dans laquelle l'élément sphérique tourne et se déplace dans une direction antéro-postérieure par rapport à la partie sphérique et à la partie de la cavité du tenon.

12. La prothèse totale de genou de l'une des revendications 1 à 11, dans laquelle le composant fémoral est adapté pour définir une première position dans laquelle l'élément sphérique est positionné dans la partie sphérique antérieure du tenon et l'élément de came n'entre pas en contact avec le tenon.

13. La prothèse totale de genou de l'une des revendications 1 à 12, dans laquelle le composant fémoral est adapté pour définir une seconde position dans laquelle l'élément sphérique est positionné dans la partie sphérique antérieure du tenon et l'élément de came entre en contact avec ce tenon.

14. La prothèse totale de genou de l'une des revendications 1 à 13, dans laquelle le composant fémoral est adapté pour définir une troisième position dans laquelle l'élément sphérique est positionné à l'intérieur de la partie de sortie postérieure et l'élément de came est en contact avec le tenon.

15. La prothèse totale de genou de l'une des revendications 1 à 14, dans laquelle le composant fémoral est adapté pour définir une quatrième position dans laquelle l'élément sphérique n'entre pas en contact avec le renfoncement du tenon et l'élément de came entre en contact avec le tenon.
